# EUROPÄISCHE PATENTSCHRIFT

(11) **EP 1 171 413 B1**
(45) Veröffentlichungstag und Bekanntmachung des Hinweises auf die Patenterteilung: **17.03.2004**
(21) Anmeldenummer: 99973824.8
(22) Anmeldetag: 26.11.1999
(51) Int. Cl.: C07C 69/44, C07C 69/80, C07C 67/08, C08K 5/11

(54) **GEMISCH VON DIESTERN DER ADIPIN- ODER PHTHALSÄURE MIT ISOMEREN NONANOLEN**
MIXTURE OF ADIPIC OR PHTHALIC ACID DIESTERS AND ISOMERIC NONANOLS
MELANGE DE DIESTERS D'ACIDE ADIPIQUE OU PHTALIQUE AVEC DES NONANOLS ISOMERIQUES

(30) Priorität: 21.04.1999 DE 19918051; 27.05.1999 DE 19924339
(43) Veröffentlichungstag der Anmeldung: 16.01.2002
(73) Patentinhaber: BASF AKTIENGESELLSCHAFT, 67056 Ludwigshafen (DE)
(72) Erfinder: THIL, Lucien, D-67117 Limburgerhof (DE); BREITSCHEIDEL, Boris, D-67117 Limburgerhof (DE); DISTELDORF, Walter, D-67157 Wachenheim (DE); ZELLER, Edgar, D-68165 Mannheim (DE); WALTER, Marc, D-67227 Frankenthal (DE); MORSBACH, Bernd, D-67069 Ludwigshafen (DE); DORNIK, Knut, D-67105 Schifferstadt (DE)
(74) Vertreter: Kinzebach, Werner, Dr.
(86) Internationale Anmeldenummer: PCT/EP1999/009208
(87) Internationale Veröffentlichungsnummer: WO 2000/063151

(56) Entgegenhaltungen:
- EP-A- 0 278 407
- WO-A-92/13818
- DE-A- 2 009 505
- DE-A- 2 855 421
- DE-A- 4 339 713

## Beschreibung

Die vorliegende Erfindung betrifft ein Gemisch von Diestern der Adipinsäure oder der Phthalsäure mit isomeren Nonanolen. Die Alkoholkomponente dieser Diester wird von einem Gemisch isomerer Nonanole gebildet.

Langkettige Alkohole, z. B. C₈-, C₉- und C₁₀-Alkohole, werden in großem Umfang zur Herstellung von Weichmachern eingesetzt. Die Alkohole werden hierzu mit Polycarbonsäuren, wie Phthalsäure oder Adipinsäure, unter Bildung der entsprechenden Ester umgesetzt. Zu den wirtschaftlich bedeutenden Vertretern zählen Adipinsäureester mit C₈-, C₉- und C₁₀-Alkoholen, z. B. Di(2-Ethylhexyl)adipat, Diisononyladipate und Diisodecyladipate, und Phthalsäureester mit C₈-, C₉- und C₁₀-Alkoholen, wie Di(2-ethylhexyl)phthalat, Diisononylphthalate und Diisodecylphthalate.

Diisononyladipate finden vor allem in Folien, Profilen, Kunstleder, Kabeln und Leitungen auf Weich-PVC-Basis Verwendung. Diisononyladipate werden insbesondere dann verwendet, wenn die Produkte bei tiefen Temperaturen eingesetzt werden sollen.

Diisononylphthalate finden vor allem in Folien, Beschichtungen und Fußbodenbelägen auf Weich-PVC-Basis Verwendung. Darüber hinaus werden sie zur Herstellung von PVC-Kabelummantelungen eingesetzt, unter anderem auch für Anwendungen bei höheren Temperaturen.

Die DE-A-20 09 505 offenbart die Verwendung von Isononanolen in Form von Bis-Isononylestern der Phthalsäure oder Adipinsäure als Weichmacher, wobei die Isononanole aus 2-Ethylhexen-1 in bekannter Weise nach der Oxosynthese durch Umsetzen mit Kohlenmonoxid und Wasserstoff bei erhöhter Temperatur und unter erhöhtem Druck in Gegenwart von Carbonylkomplexen von Metallen der VIII. Nebengruppe des Periodischen Systems und gegebenenfalls nachfolgender Hydrierung hergestellt wurden. Das 2-Ethylhexen-1 ist durch Dimerisierung von Buten-1 mit Aluminiumtrialkylen zugänglich. Die beschriebenen Bis-Isononylester sollen als Weichmacher für Polyvinylchlorid geeignet sein und sich durch geringe Flüchtigkeit, niedrige Viskosität und gute Kältefestigkeit der damit weichgemachten Polyvinylchloridmassen auszeichnen.

In der US 4,623,748 werden Dialkyladipate, unter anderem Diisononyladipate, beschrieben, die durch Umsetzung von Propylen- oder Butylen-Oligomeren aus dem Dimersol-Prozess in Gegenwart von geträgerten Tantal(V)halogeniden/oxiden als Katalysatoren, Reaktion der erhaltenen C₈-, C₉- bzw. C₁₂-Olefine zu C₉-, C₁₀- bzw. C₁₃-Alkoholen und Veresterung dieser Alkohole mit Adipinsäure hergestellt werden. Die Dialkyladipate sollen sich durch hohe Flammpunkte auszeichnen und für den Einsatz als Schmierstoffe geeignet sein.

Aus der GB 1,114,379 geht ein Gemisch isomerer Nonanole hervor, das n-Nonanol enthält und im Wesentlichen keine Alkanole mit mehr als einer Verzweigung im Kohlenstoffgerüst umfasst. Das Gemisch wird durch Hydroformylierung einer Octenfraktion, die durch Aluminiumtrialkyl-katalysierte Polymerisation von Ethylen erhalten ist, und anschließende Reduktion des Hydroformylierungsproduktes hergestellt.

In der WO 92/13818 wird die Herstellung von Diisononylphthalaten ausgehend von Butenen und gegebenenfalls Propen enthaltenden Olefingemischen durch Oligomerisierung an geträgerten Phosphorsäure-Katalysatoren bei Reaktionstemperaturen von 200 bis 235 °C zu im Wesentlichen Octene enthaltenden Olefingemischen, Hydroformylierung dieser octenhaltigen Olefingemische mit anschließender Hydrierung zu im Wesentlichen Isononanole enthaltenden Alkoholgemischen und Veresterung dieser isononanolhaltigen Alkoholgemische mit Phthalsäureanhydrid beschrieben. Die im Wesentlichen Diisononylphthalate enthaltenden Estergemische sollen als Weichmacher für PVC geeignet sein.

In der DE 28 55 421 werden Phthalate von C₉-Alkoholen beschrieben, die durch Oxo-Reaktion von C₈-Olefinen, Hydrierung des Reaktionsproduktes und Veresterung der C₉-Alkohole mit Phthalsäureanhydrid erhalten werden, wobei 3 bis 20 % der C₈-Olefine in jeder Molekülkette ein Isobutan-Skelett und weniger als 3 % der Olefine quaternären Kohlenstoff aufweisen sowie mehr als 90 % der Gesamtmenge der C₈-Olefine als n-Octene, Monomethylheptene und Dimethylhexene vorliegen. Die C₉-Phthalate sollen als Weichmacher für PVC geeignet sein.

Die EP 0 278 407 beschreibt ein C₉-Alkoholgemisch für Weichmacher, das bestimmte Anteile von Komponenten mit einem spezifizierten GC-Retentionsverhalten bezüglich bestimmter Referenzverbindungen enthält. Weichmacher auf der Basis des C₉-Alkoholgemisches sollen zu günstiger Kältebeständigkeit und guten Elektroisolationseigenschaften führen.

Für den Einsatz von Diisononylestern als Weichmacher in thermoplastischen Formmassen auf PVC-Basis ist es wichtig, dass die damit weichgemachten PVC-Compounds sowohl eine sehr hohe thermische Stabilität als auch sehr gute kälteelastische Eigenschaften zeigen. Wichtig ist ferner, dass die Weichmacher während des Gebrauchs nicht oder nur in geringem Ausmaß aus dem Weich-PVC-Compound ausschwitzen. Von den in der oben angeführten Patentliteratur beschriebenen Diisononylestern sowie von handelsüblichen Weichmachern auf der Basis von Diisononylestern wird diese Eigenschaftskombination nicht in idealer Weise erfüllt.

Der vorliegenden Erfindung liegt daher die Aufgabe zugrunde, ein Gemisch von Diestern einer Dicarbonsäure mit isomeren Nonanolen bereitzustellen, das beim Einsatz als Weichmacher in Formmassen auf PVC-Basis zu guten kälteelastischen Eigenschaften und vorzugsweise hoher thermischer Stabilität der Formmassen führt und vorzugsweise eine hohe Verträglichkeit aufweist.

Erfindungsgemäß wird diese Aufgabe gelöst durch ein Gemisch von Diestern einer Dicarbonsäure mit isomeren Nonanolen, ausgewählt unter
einem Gemisch von Diestern der Adipinsäure, in dessen in CDCl₃ aufgenommenem ¹H-NMR-Spektrum das Verhältnis der Fläche unter den Resonanzsignalen bei chemischen Verschiebungen im Bereich von 1,0 bis 2,0 ppm gegen TMS zu der Fläche unter den Resonanzsignalen bei chemischen Verschiebungen im Bereich von 0,5 bis 1,0 ppm gegen TMS zwischen 1,20 und 5,00 liegt, oder
einem Gemisch von Diestern der Phthalsäure mit isomeren Nonanolen, in dessen in CDCl₃ aufgenommenem ¹H-NMR-Spektrum das Verhältnis der Fläche unter den Resonanzsignalen bei chemischen Verschiebungen im Bereich von 1,1 bis 3,0 ppm gegen TMS zu der Fläche unter den Resonanzsignalen bei chemischen Verschiebungen im Bereich von 0,5 bis 1,1 ppm gegen TMS zwischen 1,00 und 4,00 liegt.

Im Folgenden werden die Diester der Adipinsäure mit isomeren Nonanolen auch als "Diisononyladipate", die Diester der Phthalsäure mit isomeren Nonanolen auch als "Diisononylphthalate", oder beide einfach als "Diester" bezeichnet.

Die Eigenschaften der Diisononyladipate und der Diisonylphthalate bzw. deren Einfluss auf die Eigenschaften der damit weichgemachten Formmassen werden von der Molekülstruktur bestimmt, insbesondere von der Struktur der Isononylgruppen der Alkoholkomponente. Es wurde gefunden, dass vorteilhafte kälteelastische Eigenschaften und gleichzeitig eine hohe Verträglichkeit erhalten werden, wenn die Isononylgruppen der Alkoholkomponente im Weichmacher ein bestimmtes Verhältnis von Methylen-(CH₂)- und Methyliden-(CH)-Gruppen zu Methyl-(CH₃)-Gruppen aufweisen. Die verschiedenen Gruppen sind mit Hilfe der ¹H-NMR-Spektroskopie hinreichend voneinander zu unterscheiden.

Die erfindungsgemäßen Diisononyladipate bzw. -phthalate zeichnen sich durch ein spezifisches Verhältnis von Methylen- und Methylidengruppen zu Methylgruppen im Isononylrest aus. Die Ermittlung dieses Verhältnisses erfolgt für die Zwecke der vorliegenden Erfindung mit Hilfe der ¹H-NMR-Spektroskopie an einer Lösung der Diisononyladipate bzw. -phthalate in Deuterochloroform (CDCl₃). Für die Aufnahme der Spektren werden z. B. 100 mg Substanz in 5 ml CDCl₃ gelöst und in ein NMR-Messröhrchen mit einem Durchmesser von 5 mm gefüllt. Für die vorliegenden NMR-spektroskopischen Untersuchungen wurde ein Gerät vom Typ "DPX-400" der Fa. Bruker eingesetzt. Die Spektren wurden mit einem Delay von 1 Sekunde, 32 Scans, einer Pulslänge von 3,7 µs und einer Sweep Width von 8 278,146 Hz aufgenommen. Die Resonanzsignale werden gegen die chemische Verschiebung gegen Tetramethylsilan (TMS) als internen Standard aufgezeichnet. Mit anderen handelsüblichen NMR-Geräten werden mit den gleichen Betriebsparametern vergleichbare Ergebnisse erhalten.

In den erhaltenen ¹H-NMR-Spektren der Diisononyladipate lassen sich unter den Resonanzsignalen die Signale der Methylgruppen der Isononylgruppen mit chemischen Verschiebungen von 0,5 bis 1,0 ppm von den Signalen der vereinten Methylen- und Methylidengruppen der Isononylgruppen und der Adipinsäureeinheit (innenliegende Methylengruppen) mit chemischen Verschiebungen von 1,0 bis 2,0 ppm unterscheiden. Die Quantifizierung erfolgt durch Bestimmung der Fläche unter den jeweiligen Resonanzsignalen, d. h. der vom Signal mit der Grundlinie eingeschlossenen Fläche. Handelsübliche NMR-Geräte verfügen über Vorrichtungen zur Integration der Signalflächen. Anschließend wird der Integralwert der den Methylen- und Methylidengruppen zuzuordnenden Signale im Bereich von 1,0 bis 2,0 ppm durch den Integralwert der den Methylgruppen zuzuordnenden Signale im Bereich von 0,5 bis 1,0 ppm dividiert und man erhält so ein Intensitätsverhältnis, das die Anzahl der Methylen- und Methylidengruppe im Isononylrest und in der Adipinsäureeinheit im Verhältnis zur Anzahl der Methylgruppen im Isononylrest quantitativ beschreibt.

Bei den erfindungsgemäßen Diisononyladipaten ist dieses Intensitätsverhältnis größer als 1,20, bevorzugt größer als 1,60, besonders bevorzugt größer als 1,70. Das Intensitätsverhältnis ist kleiner als 5,00, insbesondere kleiner als 2,30, besonders bevorzugt kleiner als 2,00. Vorzugsweise liegt das Verhältnis zwischen 1,60 und 2,30 und insbesondere zwischen 1,70 und 2,00.

In den erhaltenen ¹H-NMR-Spektren der Diisononylphthalate lassen sich unter den Resonanzsignalen der Isononylgruppen die Signale der Methylgruppen mit chemischen Verschiebungen von 0,5 bis 1,1 ppm von den Signalen der Methylen- und Methylidengruppen mit chemischen Verschiebungen von 1,1 bis 3,0 ppm unterscheiden. Die Quantifizierung erfolgt wie beschrieben durch Bestimmung der Fläche unter den jeweiligen Resonanzsignalen. Anschließend wird der Integralwert der den Methylen- und Methylidengruppen zuzuordnenden Signale im Bereich von 1,1 bis 3,0 ppm durch den Integralwert der den Methylgruppen zuzuordnenden Signale im Bereich von 0,5 bis 1,1 ppm dividiert und man erhält so ein Intensitätsverhältnis, das die Anzahl der Methylen- und Methylidengruppe im Isononylrest im Verhältnis zur Anzahl der Methylgruppen quantitativ beschreibt.

Bei den erfindungsgemäßen Diisononylphthalaten ist dieses Intensitätsverhältnis größer als 1,00, bevorzugt größer als 1,50, besonders bevorzugt größer als 1,55. Das Intensitätsverhältnis ist kleiner als 4,00, insbesondere kleiner als 2,00, besonders bevorzugt kleiner als 1,80. Vorzugsweise liegt das Verhältnis zwischen 1,50 und 2,00.

Die erfindungsgemäßen Diisononylphthalate zeichnen sich weiterhin dadurch aus, dass innerhalb der den Methylgruppen zuzuordnenden Signale zwischen 0,8 und 1,1 ppm im Bereich 0,7 bis 0,8 ppm ein Triplettsignal detektiert werden kann. Das Verhältnis aus dem Integralwert der Signalflächen der restlichen Signale des Methylgruppenbereichs, d. h. bei 0,8 bis 1,1 ppm, und dem Integralwert der Signalfläche des Triplettsignals bei 0,7 bis 0,8 ppm liegt dabei bei mindestens 20,0, bevorzugt bei mindestens 25,0. Es beträgt vorzugsweise höchstens 200, insbesondere höchstens 100.

Die erfindungsgemäßen Estergemische sind in einem mehrstufigen Verfahren ausgehend von einem Butene enthaltenden Kohlenwasserstoffgemisch erhältlich. In einem ersten Schritt werden die Butene zu einem Gemisch isomerer Octene dimerisiert. Das Octengemisch wird anschließend zu C₉-Aldehyden hydroformyliert und nachfolgend zu Isononanolen hydriert, welche anschließend in die Adipinsäure- bzw. Phthalsäureester umgewandelt werden. Die Herstellung von Diisononyladipaten bzw. -phthalaten durch die genannte Abfolge von Syntheseschritten ist an sich bekannt. Ein Estergemisch, das der vorstehenden Definition genügt, wird jedoch nur erhalten, wenn zumindest bei der Butendimerisierung, vorzugsweise bei der Butendimerisierung und der Hydroformylierung, spezielle, definierte Randbedingungen eingehalten werden.

So ist es bevorzugt, dass das Gemisch isomerer Octene durch Inkontaktbringen eines Butene enthaltenden Kohlenwasserstoffgemisches mit einem Nickeloxid enthaltenden heterogenen Katalysator erhalten wird. Vorzugsweise beträgt der iso-Butengehalt des Kohlenwasserstoffgemisches 5 Gew.-% oder weniger, insbesondere 3 Gew.-% oder weniger, besonders bevorzugt 2 Gew.-% oder weniger, und am meisten bevorzugt 1,5 Gew.-% oder weniger, jeweils bezogen auf den Gesamtbutengehalt. Ein geeigneter Kohlenwasserstoffstrom ist der sogenannte C₄-Schnitt, ein Gemisch aus Butenen und Butanen, das in großen Mengen aus FCC-Anlagen oder Steamcrackern zur Verfügung steht. Als besonders bevorzugter Einsatzstoff wird das sogenannte Raffinat II verwendet, bei dem es sich um einen iso-Buten-abgereicherten C₄-Schnitt handelt.

Ein bevorzugtes Einsatzmaterial enthält 50 bis 100 Gew.-%, vorzugsweise 80 bis 95 Gew.-%, Butene und 0 bis 50 Gew.-%, vorzugsweise 5 bis 20 Gew.-%, Butane. Als allgemeiner quantitativer Rahmen lässt sich folgende Zusammensetzung der Butenfraktion anführen:

| | |
|---|---|
| 1-Buten | 1 bis 99 Gew.-%, |
| cis-2-Buten | 1 bis 50 Gew.-%, |
| trans-2-Buten | 1 bis 99 Gew.-%, |
| iso-Buten | bis zu 3 Gew.-%. |

Als Katalysator kommen an sich bekannte, Nickeloxid enthaltende Katalysatoren in Betracht, wie sie z. B. von O'Connor et al. in Catalysis Today, 6, (1990) S. 329 beschrieben sind. Es können geträgerte Nickeloxidkatalysatoren verwendet werden, wobei als Trägermaterialien Kieselsäure, Tonerde, Aluminosilicate, Aluminosilicate mit Schichtstruktur und Zeolithe in Betracht kommen. Besonders geeignet sind Fällungskatalysatoren, die durch Mischen wässriger Lösungen von Nickelsalzen und Silicaten, z. B. von Natriumsilicat und Nickelnitrat, und gegebenenfalls weiteren Bestandteilen, wie Aluminiumsalzen, z. B. Aluminiumnitrat, und Calcinieren erhältlich sind.

Besonders bevorzugt sind Katalysatoren, die im Wesentlichen aus NiO, SiO₂, TiO₂ und/oder ZrO₂ sowie gegebenenfalls Al₂O₃ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserlösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE-4 339 713 verwiesen. Auf die Offenbarung dieser Druckschrift wird vollinhaltlich Bezug genommen.

Das Inkontaktbringen des Butene enthaltenden Kohlenwasserstoffgemisches mit dem Katalysator erfolgt vorzugsweise bei Temperaturen von 30 bis 280 °C, insbesondere 30 bis 140 °C und besonders bevorzugt von 40 bis 130 °C. Es erfolgt vorzugsweise bei einem Druck von 10 bis 300 bar, insbesondere von 15 bis 100 bar und besonders bevorzugt von 20 bis 80 bar. Der Druck wird dabei zweckmäßigerweise so eingestellt, dass bei der gewählten Temperatur das olefinreiche Kohlenwasserstoffgemisch flüssig oder im überkritischen Zustand vorliegt.

Geeignete Reaktionsapparaturen für das Inkontaktbringen des Kohlenwasserstoffgemisches mit dem heterogenen Katalysator sind z. B. Rohrbündelreaktoren oder Schachtöfen. Aufgrund der geringeren Investitionskosten sind Schachtöfen bevorzugt. Die Dimerisierung kann in einem einzelnen Reaktor durchgeführt werden, wobei der Oligomerisierungskatalysator in einem einzigen oder mehreren Festbetten im Reaktor angeordnet sein kann. Alternativ kann eine Reaktorkaskade aus mehreren, vorzugsweise zwei, hintereinandergeschalteten Reaktoren eingesetzt werden, wobei beim Passieren des bzw. der dem letzten Reaktor der Kaskade vorgeschalteten Reaktors bzw. Reaktoren die Butendimerisierung im Reaktionsgemisch nur bis zu einem Teilumsatz betrieben wird und der gewünschte Endumsatz erst beim Passieren des Reaktionsgemisches durch den letzten Reaktor der Kaskade erzielt wird. Die Butendimerisierung erfolgt vorzugsweise in einem adiabatischen Reaktor oder einer adiabatischen Reaktorkaskade.

Nach dem Verlassen des Reaktors bzw. des letzten Reaktors einer Kaskade werden aus dem Reaktoraustrag die gebildeten Octene und gegebenenfalls höhere Oligomere von den nicht umgesetzten Butenen und Butanen abgetrennt. Die gebildeten Oligomere können in einem nachfolgenden Vakuumfraktionierungsschritt aufgereinigt werden, wobei eine reine Octenfraktion gewonnen wird. In geringem Umfang werden bei der Butendimerisierung in der Regel auch Dodecene erhalten. Diese werden vorzugsweise vor der weiteren Umsetzung von den Octenen abgetrennt.

In einer bevorzugten Ausführungsform wird der von den gebildeten Oligomeren befreite Reaktoraustrag, der im Wesentlichen aus nicht umgesetzten Butenen und Butanen besteht, vollständig oder zum Teil zurückgeführt. Es ist bevorzugt, das Rückführverhaltnis so zu wählen, dass die Konzentration an Oligomeren im Reaktionsgemisch 35 Gew.-%, vorzugsweise 20 Gew.-%, bezogen auf das Reaktions-Kohlenwasserstoffgemisch, nicht übersteigt. Diese Maßnahme erhöht die Selektivität der Butendimerisierung bezüglich solcher Octene, die nach Hydroformylierung, Hydrierung und veresterung besonders bevorzugte Diisononyladipate und -phthalate liefern.

Die erhaltenen Octene werden im zweiten Verfahrensschritt in an sich bekannter Weise durch Hydroformylierung mit Synthesegas in um ein C-Atom verlängerte Aldehyde überführt. Die Hydroformylierung von Olefinen zur Herstellung von Aldehyden ist an sich bekannt und z. B. in J. Falbe, Hrsg.: New Synthesis with Carbon monoxide, Springer, Berlin, 1980 beschrieben. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -Komplexe eingesetzt, die unmodifiziert oder z. B. mit amin- oder mit phosphinhaltigen Verbindungen modifiziert sein können.

Im Rahmen der vorliegenden Erfindung erfolgt die Hydroformylierung vorzugsweise in Gegenwart eines Cobalt-Katalysators. Sie erfolgt vorzugsweise bei Temperaturen von 120 bis 240 °C, insbesondere 160 bis 200 °C, und unter einem Synthesegasdruck von 150 bis 400 bar, insbesondere 250 bis 350 bar. Die Hydroformylierung erfolgt vorzugsweise in Gegenwart von Wasser. Das Mischungsverhältnis von Wasserstoff zu Kohlenmonoxid im eingesetzten Synthesegas liegt vorzugsweise im Bereich von 70:30 bis 50:50, insbesondere 65:35 bis 55:45.

Das cobaltkatalysierte Hydroformylierungsverfahren kann als mehrstufiger Prozess durchgeführt werden, der folgende 4 Verfahrensstufen enthält: die Herstellung des Katalysators (Vorcarbonylierung), die Katalysatorextraktion, die Olefinhydroformylierung und die Entfernung des Katalysators aus dem Reaktionsprodukt (Entcobaltung). In der ersten Verfahrensstufe, der Vorcarbonylierung, wird ausgehend von einer wässrigen Cobaltsalzlösung, z. B. Cobaltformiat oder Cobaltacetat, durch Umsetzung mit Kohlenmonoxid und Wasserstoff der für die Hydroformylierung benötigte Katalysatorkomplex, d. h. HCo(CO)₄, hergestellt. In der zweiten Verfahrensstufe, der Katalysatorextraktion, wird der in der ersten verfahrensstufe hergestellte Cobaltkatalysator aus der wässrigen Phase mit einer organischen Phase, vorzugsweise mit dem zu hydroformylierenden Olefin, extrahiert. Bisweilen ist es zweckmäßig, neben dem Olefin die Reaktions- und Nebenprodukte der Hydroformylierung, sofern sie wasserunlöslich und unter den gewählten Reaktionsbedingungen flüssig sind, zur Katalysatorextraktion einzusetzen. Nach der Phasentrennung wird die mit dem Cobaltkatalysator beladene organische Phase der dritten Verfahrensstufe, der Hydroformylierung, zugeführt. In der vierten Verfahrensstufe, der Entcobaltung, wird die organische Phase des Reaktoraustritts von den Cobaltcarbonylkomplexen in Gegenwart von Prozesswasser, das Ameisensäure oder Essigsäure enthalten kann, durch Behandlung mit Sauerstoff oder Luft befreit. Dabei wird der Cobaltkatalysator oxidativ zerstört und die erhaltenen Cobaltsalze in die wässrige Phase zurückextrahiert. Die erhaltende wässrige Cobaltsalzlösung aus der Entcobaltung wird in die erste Verfahrensstufe, die Vorcarbonylierung, zurückgeführt. Das erhaltene rohe Hydroformylierungsprodukt kann unmittelbar der Hydrierung zugeführt werden. Alternativ kann daraus in üblicher weise, z. B. destillativ, eine C₉-Fraktion isoliert werden, die der Hydrierung zugeführt wird.

Die Bildung des Cobaltkatalysators, die Extraktion des Cobaltkatalysators in die organische Phase und die Hydroformylierung der Olefine kann auch in einem einstufigen Prozess im Hydroformylierungsreaktor durchgeführt werden.

Verwendbare Cobaltverbindungen sind beispielsweise Cobalt(II)-chlorid, Cobalt(II)-nitrat, deren Amin- oder Hydratkomplexe, Cobaltcarboxylate, wie Cobaltformiat, Cobaltacetat, Cobaltethylhexanoat, Cobaltnaphthanoat, sowie der Cobaltcaprolactamatkomplex. Unter den Hydroformylierungsbedingungen bilden sich in situ die katalytisch aktiven Cobaltverbindungen in Form von Cobaltcarbonylen. Es können auch die Carbonylkomplexe des Cobalts, wie Dicobaltoctacarbonyl, Tetracobaltdodecacarbonyl und Hexacobalthexadecacarbonyl eingesetzt werden.

Das bei der Hydroformylierung erhaltene Aldehydgemisch wird zu primären Alkoholen reduziert. Im Allgemeinen erfolgt eine teilweise Reduktion bereits unter den Hydroformylierungsbedingungen, wobei die Hydroformylierung auch so gesteuert werden kann, dass eine im Wesentlichen vollständige Reduktion erfolgt. In der Regel wird jedoch das erhaltene Hydroformylierungsprodukt in einem weiteren Verfahrensschritt mit Wasserstoffgas oder einem Wasserstoff enthaltendem Gasgemisch hydriert. Die Hydrierung findet in der Regel in Gegenwart eines heterogenen Hydrierkatalysators statt. Als Hydrierkatalysator kann ein beliebiger zur Hydrierung von Aldehyden zu primären Alkoholen geeigneter Katalysator verwendet werden. Beispiele geeigneter handelsüblicher Katalysatoren sind Kupferchromit, Cobalt, Cobaltverbindungen, Nickel, Nickelverbindungen, die gegebenenfalls geringe Mengen Chrom oder andere Promotoren enthalten, und Gemische von Kupfer, Nickel und/oder Chrom. Die Nickelverbindungen liegen im Allgemeinen geträgert auf Trägermaterialien, wie Tonerde oder Kieselgur, vor. Weiterhin können Edelmetall enthaltende Katalysatoren, wie Platin oder Palladium, verwendet werden.

Die Hydrierung kann geeigneterweise in Rieselfahrweise erfolgen, wobei das zu hydrierende Gemisch und das Wasserstoffgas bzw. das wasserstoffhaltige Gasgemisch z. B. im Gleichstrom über ein fest angeordnetes Bett des Hydrierkatalysators geleitet werden.

Die Hydrierung findet vorzugsweise bei einer Temperatur von 50 bis 250 °C, insbesondere 100 bis 150 °C, und einem Wasserstoffdruck von 50 bis 350 bar, insbesondere 150 bis 300 bar, statt. Aus dem bei der Hydrierung erhaltenen Reaktionsaustrag kann die gewünschte Isononanolfraktion durch fraktionierte Destillation von den C₈-Kohlenwasserstoffen und höhersiedenden Produkten abgetrennt werden.

Die Isononanole werden anschließend in einem weiteren Verfahrensschritt in an sich bekannter weise durch Veresterung mit Adipin-oder Phthalsäure oder einem Derivat davon in die Diester der Adipin- oder Phthalsäure mit den isomeren Nonanolen überführt. Zur Herstellung der erfindungsgemäßen Phthalsäureester ist die Verwendung von Phthalsäureanhydrid bevorzugt. Vorzugsweise wird das oben beschriebene Gemisch isomerer Nonanole im Überschuss mit der jeweiligen Dicarbonsäure oder dem Derivat davon, insbesondere einem molaren Überschuss von 5 bis 30 %, und vorzugsweise in Gegenwart eines Acylierungskatalysators, wie eines Dialkyltitanats, z. B. Isopropylbutyltitanat, oder einer Säure, wie Methansulfonsäure oder Schwefelsäure, umgesetzt.

Die Umsetzung mit der Dicarbonsäure oder einem Dicarbonsäurederivat findet im Allgemeinen bei Reaktionstemperaturen von 150 bis 300 °C, vorzugsweise 200 bis 250 °C statt. Bei einer geeigneten Ausführungsform wird während der Umsetzung ein Inertgas, wie Stickstoff, in das Reaktionsgemisch eingeperlt und das gebildete Reaktionswasser fortlaufend mit dem Inertgasstrom aus dem Reaktionsgemisch entfernt. Nach beendeter Umsetzung wird aus dem Reaktionsgemisch das erfindungsgemäße Gemisch von Diestern der Adipin- bzw. Phthalsäure mit isomeren Nonanolen isoliert. Dies kann z. B. durch Abdestillieren des Isononanol-Überschusses im Vakuum, Neutralisieren des Roh-Diesters mit einer wässrigen Lauge, wie Natronlauge, unter Bildung eines Zweiphasengemisches, Abtrennen der wässrigen Phase, Waschen der organischen Phase, geschehen. Zur weiteren Reinigung wird der neutralisierte und gewaschene Diester vorzugsweise mit Wasserdampf bei erhöhter Temperatur unter Vakuum ausgedämpft. Der gereinigte Diester kann dann bei erhöhter Temperatur im Vakuum durch Durchleiten eines N₂-Stroms getrocknet, und gegebenenfalls durch Inkontaktbringen mit einem Adsorptionsmittel, wie Aktivkohle oder Bleicherde, weiter gereinigt werden.

Die auf diese Weise hergestellten erfindungsgemäßen Diisononyladipate besitzen eine Dichte zwischen 0,900 und 0,940 g/cm³, bevorzugt 0,910 und 0,930 g/cm³, besonders bevorzugt 0,918 und 0,922 g/cm³, eine Viskosität zwischen 15,0 und 25,0 mPa·s, bevorzugt zwischen 16,0 und 22,0 mPa·s, besonders bevorzugt 17,0 und 20,0 mPa·s, und einen Brechungsindex n_{D}²⁰ zwischen 1,445 und 1,455, bevorzugt 1,447 und 1,453, besonders bevorzugt 1,448 und 1,452.

Die auf diese weise hergestellten erfindungsgemäßen Diisononylphthalate besitzen im Allgemeinen eine Dichte zwischen 0,950 und 0,990 g/cm³, vorzugsweise 0,960 und 0,980 g/cm³, insbesondere zwischen 0,967 und 0,973 g/cm³, eine Viskosität zwischen 60,0 und 110,0 mPa·s, vorzugsweise zwischen 63,0 und 80,0 mPa·s, insbesondere zwischen 65,0 und 75,0 mPa·s, und einen Brechungsindex n_{D}²⁰ zwischen 1,470 und 1,495, vorzugsweise 1,476 und 1,490, insbesondere zwischen 1,480 und 1,486.

Die erfindungsgemäßen Gemische von Diestern eignen sich bevorzugt als Weichmacher für die Herstellung von thermoplastischen Formmassen, insbesondere für Formmassen auf PVC-Basis.

Dabei eignen sich die erfindungsgemäßen Diisononyladipate insbesondere für die Herstellung von Weich-PVC-Compounds, die sehr gute kälteelastische Eigenschaften und gleichzeitig eine hohe Verträglichkeit des Weichmachers aufweisen. Die erfindungsgemäßen Diisononylphthalate sind besonders geeignet zur Herstellung von Weich-PVC-Compounds, die sich durch eine hohe thermische Stabilität und gleichzeitig sehr gute kälteelastische Eigenschaften auszeichnen.

Für die Herstellung und Untersuchung der unter Einsatz der erfindungsgemäßen Diester hergestellten Weich-PVC-Compounds wird bevorzugt folgende Methode verwendet:

Zunächst wird ein Gemisch hergestellt aus PVC-Pulver, bevorzugt PVC-Pulver, das nach dem Suspensionsverfahren hergestellt wurde, dem erfindungsgemäßen Diester als Weichmacher sowie gegebenenfalls weiteren Zusätzen, wie Stabilisatoren, Gleitmitteln, Füllstoffen, Pigmenten, Farbstoffen, Flamminhibitoren, Lichtstabilisatoren, Antistatika, Treibmitteln, Biostabilisatoren. Dieses Gemisch wird anschließend auf einem Mischwalzwerk plastifiziert und zu einem sogenannten Walzfell gewalzt. Das walzfell wird anschließend zu einer Weich-PVC-Folie verpresst, an der dann die anwendungstechnischen Untersuchungen durchgeführt werden.

Die Charakterisierung der kälteelastischen Eigenschaften der Weich-PVC-Compounds erfolgt vorzugsweise mit Hilfe der "Kältebruchtemperatur". Unter der Kältebruchtemperatur versteht man die Temperatur, bei der ein Weich-PVC-Compound in der Kälte bei mechanischer Belastung erste optisch sichtbare Beschädigungen aufweist. Die Bestimmung der Kältebruchtemperatur erfolgt nach DIN 53372. Ein weiteres Kriterium zur Charakterisierung der kälteelastischen Eigenschaften stellt die "Torsionssteifheit" dar. Unter der Torsionssteifheit versteht man die Temperatur, bei der ein Weich-PVC-Compound bei Anwendung einer definierten Kraft um einen bestimmten Winkel verdreht werden kann. Die Bestimmung der Torsionssteifheit erfolgt nach DIN 53447.

Die Charakterisierung der thermischen Stabilität von weich-PVC-Compounds erfolgt vorzugsweise mit Hilfe der sog. "HCl-Reststabilität". Darunter versteht man die Zeitspanne, innerhalb der das Weich-PVC-Compound bei einer Temperatur von 200 °C noch keine Zersetzung unter Abspaltung von HCl zeigt. Die Bestimmung der HCl-Reststabilität erfolgt nach der VDE-Norm 0472, § 614.

Die Verträglichkeit eines Weichmachers in einem Weich-PVC-Compound wird bestimmt, indem das Weich-PVC-Compound bei einer Temperatur von 70 °C und 100 % relativer Luftfeuchtigkeit über einen längeren Zeitraum gelagert und der Gewichtsverlust des Weich-PVC-Compounds infolge Ausschwitzen des Weichmachers nach bestimmten Zeitintervallen durch Auswiegen ermittelt wird. Die Untersuchung der Verträglichkeit erfolgt nach folgender Arbeitsvorschrift:

### Zweck der Untersuchung

Die Prüfung dient zur quantitativen Messung der Verträglichkeit von Weichmachern in Weich-PVC-Rezepturen. Sie wird bei erhöhter Temperatur (70 °C) und Luftfeuchte (100 % rel. H.) durchgeführt. Die erhaltenen Daten werden gegen die Lagerzeit ausgewertet.

### Probekörper

Zur Prüfung werden Prüfkörper (Folien) mit einer Größe von 75 x 110 x 0,5 mm verwendet. Die Folien werden an der Breitseite gelocht, beschriftet (Lötkolben) und gewogen.

### Prüfgeräte

Heraeus-Trockenschrank bei 70 °C, Analysenwaage, Temperaturmessgerät Testotherm mit Fühler für Innenraummessung im Trockenschrank.

### Durchführung

Es wird die Temperatur im Innenraum des Trockenschrankes auf 70 °C eingestellt. Die fertigen gewogenen Folien werden auf ein Drahtgestell aufgehängt und in eine Glaswanne, die ca. 5 cm mit Wasser (VE-Wasser) gefüllt ist, hineingestellt. Zu beachten ist, dass die Folien sich gegenseitig nicht berühren. Die Unterkanten der Folien dürfen nicht ins Wasser hängen. Die Glaswanne wird mit einer PE-Folie wasserdampfdicht verschlossen, damit der später in der Glaswanne entstehende Wasserdampf nicht entweichen kann. Der Wasserstand in den Glasbecken wird täglich kontrolliert und eventuell fehlendes Wasser wird ersetzt.

### Lagerzeit

Im Tagesrhythmus werden jeweils zwei Folien der Glaswanne entnommen und eine Stunde lang frei hängend an der Luft klimatisiert. Danach werden die Folien mit Methanol oberflächlich gereinigt. Anschließend werden die Folien für 16 h bei 70 °C in einem Trokkenschrank mit Zwangskonvektion frei hängend getrocknet. Nach Entnahme aus dem Trockenschrank werden die Folien eine Stunde lang frei hängend klimatisiert und anschließend gewogen. Angegeben wird jeweils der arithmetische Mittelwert der Gewichtsverluste der Folien.

Die Erfindung ist anhand nachfolgender Beispiele näher erläutert.

### Beispiel 1:

### Herstellung eines erfindungsgemäßen Diisononyladipates:

### Verfahrensschritt 1 (Butendimerisierung):

Die Butendimerisierung wurde in einem adiabatischen Reaktor bestehend aus zwei Teilreaktoren (Länge: jeweils 4 m, Durchmesser: jeweils 80 cm) mit Zwischenkühlung bei 30 bar kontinuierlich durchgeführt. Als Einsatzprodukt wurde ein Raffinat II mit folgender Zusammensetzung verwendet:

| | |
|---|---|
| i-Butan | 2 Gew.-% |
| n-Butan | 10 Gew.-% |
| i-Buten | 2 Gew.-% |
| Buten-1 | 32 Gew.-% |
| Buten-2-trans | 37 Gew.-% |
| Buten-2-cis | 17 Gew.-% |

Als Katalysator diente ein Material gemäß DE 4 339 713, bestehend aus 50 Gew.-% NiO, 12,5 Gew.-% TiO₂, 33,5 Gew.-% SiO₂ und 4 Gew.-% Al₂O₃ in Form von 5 x 5 mm-Tabletten. Die Umsetzung wurde mit einem Durchsatz von 0,375 kg Raffinat II/1 Katalysator·h einem Rückführverhältnis von unumgesetztem C₄-Kohlenwasserstoffen zu frischem Raffinat II von 3, einer Eintrittstemperatur am ersten Teilreaktor von 38 °C und einer Eintrittstemperatur am zweiten Teilreaktor von 60 °C durchgeführt. Der Umsatz bezogen auf die im Raffinat II enthaltenen Butene lag bei 83,1 %; die Selektivität zu den gewünschten Oktenen betrug 83,3 %. Durch fraktionierte Destillation des Reaktoraustrages wurde die Oktenfraktion von unumgesetztem Raffinat II und den Hochsiedern abgetrennt.

### Verfahrensschritt 2 (Hydroformylierung und anschließende Hydrierung):

750 g des in Verfahrensschritt 1 hergestellten Oktengemisches wurden diskontinuierlich in einem Autoklav mit 0,13 Gew.-% Dicobaltoktacarbonyl Co₂(CO)₈ als Katalysator unter Zusatz von 75 g Wasser bei 185 °C und unter einem Synthesegasdruck von 280 bar bei einem Mischungsverhältnis von H₂ zu CO von 60/40 5 Stunden umgesetzt. Der Verbrauch an Synthesegas, zu erkennen an einem Druckabfall im Autoklaven wurde durch Nachpressen ergänzt. Nach dem Entspannen des Autoklaven wurde der Reaktionsaustrag mit 10 gew.-%iger Essigsäure durch Einleiten von Luft oxidativ vom Cobaltkatalysator befreit und die organische Produktphase mit Raney-Nickel bei 125 °C und einem Wasserstoffdruck von 280 bar 10 h hydriert. Durch fraktionierte Destillation des Reaktionsaustrages wurde die Isononanolfraktion von den C₈-Paraffinen und den Hochsiedern abgetrennt.

### Verfahrensschritt 3 (Veresterung):

Im dritten Verfahrensschritt wurden 865,74 g der in Verfahrensschritt 2 erhaltene Isononanolfraktion (20 % molarer Überschuss bezogen auf Adipinsäure) mit 365,25 g Adipinsäure und 0,42 g Isopropylbutyltitanat als Katalysator in einem 2 l-Autoklaven unter N₂-Einperlung (10 l/h) bei einer Rührgeschwindigkeit von 500 U/min und einer Reaktionstemperatur von 230 °C umgesetzt. Das gebildete Reaktionswasser wurde fortlaufend mit dem N₂-Strom aus dem Reaktionsgemisch entfernt. Die Reaktionszeit betrug 180 min. Anschließend wurde der Isononanol-Überschuss bei einem Vakuum von 50 mbar abdestilliert. 1 000 g des Roh-Diisononyladipates wurden mit 150 ml 0,5 %iger Natronlauge durch 10-minütiges Rühren bei 80 °C neutralisiert. Es bildete sich ein Zwei-Phasen-Gemisch mit einer oberen organischen Phase und einer unteren wässrigen Phase (Ablauge mit hydrolysiert Katalysator). Die wässrige Phase wurde abgetrennt und die organische Phase zweimal mit 200 ml H₂O nachgewaschen. Zur weiteren Reinigung wurde das neutralisierte und gewaschene Diisononyladipat mit Wasserdampf bei 180 °C und 50 mbar Vakuum zwei Stunden lang ausgedämpft. Das gereinigte Diisononyladipat wurde dann 30 min bei 150 °C/50 mbar mittels Durchleiten eines N₂-Stroms (2 l/h) getrocknet, anschließend mit Aktivkohle 5 min verrührt und über eine Nutsche mit Filterhilfsmittel Supra-Theorit 5 abgesaugt (Temperatur 80 °C).

Das so erhaltene Diisononyladipat besitzt eine Dichte von 0,920 g/cm³, eine Viskosität von 19,2 mPa·s, einen Brechungsindex n_{D}²⁰ von 1,4500, eine Säurezahl von 0,03 mg KOH/g, einen Wassergehalt von 0,02 % und eine Reinheit nach GC von 99,86 %.

### ¹H-NMR-spektroskopische Charakterisierung des erfindungsgemäßen Diisononyladipates:

Das Integral über die Flächen der Signalgruppen im Bereich der Methylen- und Methylidengruppen (einschließlich der innenliegenden Methylengruppen der Adipinsäurekomponente) von 1,0 bis 2,0 ppm ergibt einen Wert von 576,32, das Integral über die Flächen der Signalgruppen im Bereich der Methylgruppen von 0,5 bis 1,0 ppm ergibt einen Wert von 329,47. Entsprechend resultiert ein Intensitätsverhältnis von 576,32/329,47 = 1,75.

### Herstellung und Prüfung eines Weich-PVC-Compounds unter Einsatz des erfindungsgemäßen Diisononyladipates:

150 g Suspensions-PVC vom Typ "Vinoflex S 7114"; 75 g des erfindungsgemäßen Diisononyladipates und 3 g Ba/Zn-Stabilisator vom Typ "Lankromark LZB 753" werden mit einem Handmixer bei Raumtemperatur vermischt. Die Mischung wird anschließend auf einem dampfbeheizten Labormischwalzwerk (Fa. Collin, Typ "150") plastifiziert und zu einem Walzfell verarbeitet. Die Temperatur der beiden Walzen beträgt jeweils 170 °C; die Drehzahlen liegen bei 15 Umdrehungen/min (vordere Walze) und 12 Umdrehungen/min (hintere Walze); die Walzzeit beträgt 5 Minuten. Man erhält so ein Walzfell mit einer Dicke von 0,55 mm. Das abgekühlte Walzfell wird anschließend bei einer Temperatur von 180 °C und einem Druck von 220 bar innerhalb 400 s auf einer Presse vom Typ "400 P" der Fa. Collin zu einer Weich-PVC-Folie mit einer Dicke von 0,50 mm verpresst.

An dieser Weich-PVC-Folie wird anschließend die Kältebruchtemperatur (nach DIN 53372), die Torsionssteifheit (nach DIN 53447) und die Verträglichkeit (nach obiger Arbeitsvorschrift) bestimmt. Die Ergebnisse sind in Tabelle 1 zusammen mit dem Intensitätsverhältnis aus dem ¹H-NMR-Spektrum zusammengestellt.

### Vergleichsbeispiel 1:

Zum vergleich wurde ein kommerziell verfügbares Diisononyladipat vom Typ "Exxon Jayflex DINA" ¹H-NMR-spektroskopisch untersucht und ein weich-PVC-Compound unter Einsatz dieses Diisononyladipates hergestellt und geprüft.

Im ¹H-NMR-Spektrum von Exxon Jayflex DINA ergibt sich für das Integral über die Flächen der Signalgruppen im Bereich der Methylen- und Methylidengruppen (einschließlich der innenliegenden Methylengruppen der Adipinsäurekomponente) von 1,0 bis 2,0 ppm ein Wert von 517,16, das Integral über die Flächen Signalgruppen im Bereich Methylgruppen von 0,5 bis 1,0 ppm ergibt einen Wert von 453,95. Entsprechend resultiert ein Intensitätsverhältnis von 517,16/453,95 = 1,14.

In Analogie zu Beispiel 1 wurde unter Einsatz des kommerziell verfügbaren Diisononyladipates Exxon Jayflex DINA eine Weich-PVC-Folie hergestellt und an dieser Folie die Kältebruchtemperatur (nach DIN 53372), die Torsionssteifheit (nach DIN 53447) und die Verträglichkeit (nach obiger Arbeitsvorschrift) bestimmt. Die Ergebnisse sind in Tabelle 1 zusammen mit dem Intensitätsverhältnis aus dem ¹H-NMR-Spektrum zusammengestellt.

**Tabelle 1**

| Diisononyladipat | Beispiel 1 | Vergleichsbeispiel 1 "Exxon Jayflex DINA" |
|---|---|---|
| Intensitätsverhältnis der Signalgruppen bei 1,0-2,0 ppm (CH₂-, CH-Gruppen) und 0,5-1,0 ppm (CH₃-Gruppen) im ¹H-NMR-Spektrum | 1,75 | 1,14 |
| Kältebruchtemperatur (°C) des Weich-PVC-Compounds | -64 | -56 |
| Torsionssteifheit (°C) des weich-PVC-Compounds | -55 | -49 |
| Verträglichkeitsprüfung bei 70 °C und 100 % relativer Luftfeuchtigkeit | | |
| Gewichtsverlust des Weich-PVC-Compounds (Gew.-%) | | |
| Nach 1 d | 0,11 | 0,22 |
| Nach 3 d | 0,27 | 0,33 |
| Nach 7 d | 0,57 | 0,69 |
| Nach 14 d | 0,80 | 0,88 |
| Nach 28 d | 1,00 | 1,07 |

Das erfindungsgemäße Diisononyladipat (Beispiel 1) unterscheidet sich hinsichtlich des Intensitätsverhältnisses der Signalgruppen bei 1,0 bis 2,0 ppm (CH₂-, CH-Gruppen) und 0,5 bis 1,0 ppm (CH₃-Gruppen) deutlich von dem kommerziell verfügbaren Diisononyladipat. Das Intensitätsverhältnis liegt beim Diisononyladipat von Beispiel 1 bei 1,75 im Vergleich zu 1,14 bei Exxon Jayflex DINA.

Das unter Einsatz des erfindungsgemäßen Diisononyladipates aus Beispiel 1 hergestellte Weich-PVC-Compound zeigt mit einer Kältebruchtemperatur von -64 °C und einer Torsionssteifheit von -55 °C deutlich niedrigere Werte als das auf Basis von Exxon Jayflex DI-NA hergestellte Compound (Kältebruchtemperatur -56 °C, Torsionssteifheit -49 °C). Das erfindungsgemäße Diisononyladipat bewirkt somit deutlich bessere kälteelastische Eigenschaften des Weich-PVC-Compounds im Vergleich zu Exxon Jayflex DINA.

Gleichzeitig zeigt das unter Einsatz des erfindungsgemäßen Diisononyladipates aus Beispiel 1 hergestellte Weich-PVC-Copmound eine signifikant bessere Verträglichkeit des Weichmachers gegenüber dem auf Basis von Exxon Jayflex DINA hergestellten Compound. Beispielsweise beträgt der Gewichtsverlust infolge Ausschwitzen des Weichmachers bei dem Compound in Beispiel 1 nach 7 d nur 0,57 Gew.-%, während der entsprechende Wert des Compounds des Vergleichsbeispiels bei 0,68 Gew.-% liegt. Dies belegt, dass mit den erfindungsgemäßen Diisononyladipaten Weich-PVC-Compounds mit sehr guten kälteelastischen Eigenschaften und gleichzeitig sehr guter Verträglichkeit des Weichmachers im Weich-PVC-Compound erhalten werden.

### Beispiel 2

### Herstellung eines erfindungsgemäßen Diisonoylphthalats:

Die Herstellung erfolgte durch Veresterung von 865,74 g der gemäß Beispiel 1, Verfahrensschritte 1 und 2, erhaltenen Isononanolfraktion (20 % Überschuss bezüglich Phthalsäureanhydrid) mit 370,30 g Phthalsäureanhydrid und 0,42 g Isopropylbutyltitanat als Katalysator in einem 2 l-Autoklaven unter N₂-Einperlung (10 l/h) bei einer Rührgeschwindigkeit von 500 U/min und einer Reaktionstemperatur von 230 °C umgesetzt. Das gebildete Reaktionswasser wurde fortlaufend mit dem N₂-Strom aus dem Reaktionsgemisch entfernt. Die Reaktionszeit betrug 180 min. Anschließend wurde der Isononanol-Überschuss bei einem Vakuum von 50 mbar abdestilliert. 1 000 g des Roh-Diisononylphthalates wurden mit 150 ml 0,5 %-iger Natronlauge durch 10 minütiges Rühren bei 80 °C neutralisiert. Es bildete sich ein Zwei-Phasen-Gemisch mit einer oberen organischen Phase und einer unteren wässrigen Phase (Ablauge mit hydrolysiertem Katalysator). Die wässrige Phase wurde abgetrennt und die organische Phase zweimal mit 200 ml H₂O nachgewaschen. Zur weiteren Reinigung wurde das neutralisierte und gewaschene Diisononylphthalat mit Wasserdampf bei 180 °C und 50 mbar Vakuum 2 h ausgedämpft. Das gereinigte Diisononylphthalat wurde dann 30 min bei 150 °C/50 mbar mittels Durchleiten eines N₂-Stroms (2 l/h) getrocknet, anschließend mit Aktivkohle 5 min verrührt und über eine Nutsche mit Filterhilfsmittel Supra-Theorit 5 abgesaugt (Temperatur 80 °C).

Das so erhaltene Diisononylphthalat besitzt eine Dichte von 0,973 g/cm³, eine Viskosität von 73,0 mPa·s, einen Brechungsindex n_{D}²⁰ von 1,4853, eine Säurezahl von 0,03 mg KOH/g, einen Wassergehalt von 0,03 % und eine Reinheit nach GC von 99,83 %.

### ¹H-NMR-spektroskopische Charakterisierung des erfindungsgemäßen Diisononylphthalates:

Das ¹H-NMR-Spektrum des Diisononylphthalates aus Beispiel 1 wurde mit einem NMR-Gerät vom Typ "DPX-400" der Fa. Bruker aufgenommen. Das Integral über die Flächen der Signalgruppen im Bereich der Methylen- und Methylidengruppen von 1,1 bis 3,0 ppm ergibt einen Wert von 125,5, das Integral über die Flächen der Signalgruppen im Bereich der Methylgruppen von 0,5 bis 1,1 ppm ergibt einen Wert von 75,5. Entsprechend resultiert ein Intensitätsverhältnis von 125,5/75,5 = 1,66.

Bei höherer Auflösung erkennt man die Signale des Methylgruppenbereichs bei 0,8 bis 1,1 ppm mit einem Integralwert von 1 000 über die Signalflächen und ein Triplettsignal bei 0,7 bis 0,8 ppm mit einem Integralwert von 39,493 über die Signalfläche. Es resultiert ein Intensitätsverhältnis von 1 000/39,493 = 25,3.

### Herstellung und Prüfung eines Weich-PVC-Compounds unter Einsatz des erfindungsgemäßen Diisononylphthalates:

150 g Suspensions-PVC vom Typ "Vinoflex S 7114", 100 g des erfindungsgemäßen Diisononylphthalates und 3 g Ba/Zn-Stabilisator vom Typ "Lankromark LZB 753" werden mit einem Handmixer bei Raumtemperatur vermischt. Die Mischung wird anschließend auf einem dampfbeheizten Labormischwalzwerk (Fa. Collin, Typ "150") plastifiziert und zu einem Walzfell verarbeitet. Die Temperatur der beiden Walzen beträgt jeweils 170 °C; die Drehzahlen liegen bei 15 Upm (vordere Walze) und 12 Upm (hintere Walze); die Walzzeit beträgt 5 Minuten. Man erhält so ein Walzfell mit einer Dicke von 0,55 mm. Das abgekühlte Walzfell wird anschließend bei einer Temperatur von 180 °C und einem Druck von 220 bar innerhalb 400 s auf einer Presse vom Typ "400 P" der Fa. Collin zu einer Weich-PVC-Folie mit einer Dicke von 0,50 mm verpresst.

An dieser Weich-PVC-Folie wird anschließend die HCl-Reststabilität (nach VDE-Norm 0472, § 614) und die Kältebruchtemperatur (nach DIN 53372) bestimmt. Die Ergebnisse sind in Tabelle 2 zusammen mit den Intensitätsverhältnissen aus dem ¹H-NMR-Spektrum zusammengestellt.

### Vergleichsbeispiele 2 und 3:

Zum Vergleich wurden zwei im Handel erhältliche Diisononylphthalate vom Typ "Exxon Jayflex DINP" (Vergleichsbeispiel 2) und "BASF Palatinol DN" (Vergleichsbeispiel 3) wie oben ¹H-NMR-spektroskopisch untersucht und Weich-PVC-Compounds unter Einsatz dieser Diisononylphthalate hergestellt und geprüft.

Im ¹H-NMR-Spektrum von Exxon Jayflex DINP weist das Integral über die Flächen der Signalgruppen im Bereich der Methylen- und Methylidengruppen von 1,1 bis 3,0 ppm einen Wert von 95, das Integral über die Flächen der Signalgruppen im Bereich der Methylgruppen von 0,5 bis 1,1 ppm einen Wert von 106 auf. Entsprechend resultiert ein Intensitätsverhältnis von 95/106 = 0,90. Ein Triplettsignal im Bereich 0,7 bis 0,8 ppm konnte nicht detektiert werden.

Im ¹H-NMR-Spektrum von BASF Palatinol DN weist das Integral über die Flächen der Signalgruppen im Bereich der Methylen- und Methylidengruppen von 1,1 bis 3,0 ppm einen Wert von 70, das Integral über die Flächen der Signalgruppen im Bereich der Methylgruppen von 0,5 bis 1,1 ppm einen Wert von 131,5 auf. Entsprechend resultiert ein Intensitätsverhältnis von 70/131,5 = 0,53. Ein Triplettsignal im Bereich 0,7 bis 0,8 konnte nicht detektiert werden.

In Analogie zu Beispiel 2 wurden unter Einsatz der kommerziell verfügbaren Diisononylphthalate Exxon Jayflex DINP und BASF Palatinol DN Weich-PVC-Folien hergestellt und an diesen Folien die HCl-Reststabilität (nach VDE-Norm 0472, § 614) und die Kältebruchtemperatur (nach DIN 53372) bestimmt. Die Ergebnisse sind in Tabelle 2 zusammen mit den Intensitätsverhältnissen aus dem ¹H-NMR-Spektrum zusammengestellt.

**Tabelle 2**

| Diisononylphthalat | Beispiel 2 | Vergleichsbeispiel 2 "Exxon Jayflex DINP" | Vergleichsbeispiel 3 "BASF Palatinol DN" |
|---|---|---|---|
| Intensitätsverhältnis der Signalgruppen bei 1,1-3,0 ppm (CH₂-, CH-Gruppen) und 0,5-1,1 ppm (CH₃-Gruppen) im ¹H-NMR-Spektrum | 1,66 | 0,90 | 0,53 |
| Intensitätsverhältnis der Signalgruppen bei 0,8-1,1 ppm und der Signalgruppe (Triplett) bei 0,7-0,8 ppm im ¹H-NMR-Spektrum | 25,3 | - | - |
| Kältebruchtemperatur (°C) des Weich-PVC-Compounds | -42 | -31 | -23 |
| HCl-Reststabilität bei 200 °C (min) des Weich-PVC-Compounds | 18 | 13,8 | 14,4 |

Aus den Daten in Tabelle 2 geht eindeutig hervor, dass sich das erfindungsgemäße Diisononylphthalat (Beispiel 2) hinsichtlich des Intensitätsverhältnisses der Signalgruppen bei 1,1 bis 3,0 ppm (CH₂-, CH-Gruppen) und 0,5 bis 1,1 ppm (CH₃-Gruppen) deutlich von den im Handel erhältlichen Diisononylphthalaten unterscheidet. Das Intensitätsverhältnis liegt bei Beispiel 2 bei 1,66 im Vergleich zu 0,90 bei Exxon Jayflex DINP und 0,53 bei BASF Palatinol DN. Die im Handel erhältlichen Diisononylphthalate weisen darüber hinaus im ¹H-NMR-Spektrum kein Triplettsignal bei 0,7 bis 0,8 ppm auf.

Das unter Einsatz des erfindungsgemäßen Diisononylphthalates aus Beispiel 2 hergestellte Weich-PVC-Compound zeigt mit einer Kältebruchtemperatur von -42 °C einen deutlich niedrigeren Wert als die auf Basis von Exxon Jayflex DINP und BASF Palatinol DN (Vergleichsbeispiele 2 und 3) hergestellten Compounds (Kältebruchtemperaturen -31 °C bzw. -23 °C). Gleichzeitig zeigt das unter Einsatz des erfindungsgemäßen Diisononylphthalates aus Beispiel 2 hergestellte Weich-PVC-Compound mit einer HCl-Reststabilität von 18 min einen deutlich höheren Wert als die auf Basis von Exxon Jayflex DINP und BASF Palatinol DN hergestellten Compounds (HCl-Reststabilitäten 13,8 min bzw. 14,4 min). Dies belegt, dass mit den erfindungsgemäßen Diisononylphthalaten Weich-PVC-Compounds mit hoher thermischer Stabilität und gleichzeitig sehr guten kälteelastischen Eigenschaften erhalten werden.

## Patentansprüche

1. Gemisch von Diestern einer Dicarbonsäure mit isomeren Nonanölen, ausgewählt unter
einem Gemisch von Diestern der Adipinsäure, in dessen in CDCl₃ aufgenommenem ¹H-NMR-Spektrum das Verhältnis der Fläche unter den Resonanzsignalen bei chemischen Verschiebungen im Bereich von 1,0 bis 2,0 ppm gegen TMS zu der Fläche unter den Resonanzsignalen bei chemischen Verschiebungen im Bereich von 0,5 bis 1,0 ppm gegen TMS zwischen 1,20 und 5,00 liegt,
oder
einem Gemisch von Diestern der Phthalsäure, in dessen in CDCl₃ aufgenommenem ¹H-NMR-Spektrum das Verhältnis der Fläche unter den Resonanzsignalen bei chemischen Verschiebungen im Bereich von 1,1 bis 3,0 ppm gegen TMS zu der Fläche unter den Resonanzsignalen bei chemischen Verschiebungen im Bereich von 0,5 bis 1,1 ppm gegen TMS zwischen 1,00 und 4,00 liegt.

2. Gemisch nach Anspruch 1, erhältlich durch Veresterung eines Gemisches isomerer Nonanole, das durch Hydroformylierung und Hydrierung eines Gemisches isomerer Octene erhältlich ist, mit einer Dicarbonsäure, ausgewählt unter Adipinsäure und Phthalsäure oder einem Derivat davon, wobei das Gemisch isomerer Octene durch Inkontaktbringen eines Butene enthaltenden Kohlenwasserstoffgemisches mit einem Nickeloxid enthaltenden heterogenen Katalysator erhältlich ist.

3. Gemisch nach Anspruch 2, **dadurch gekennzeichnet, dass** das Kohlenwasserstoffgemisch 5 Gew.-% oder weniger, bezogen auf den Gesamtbutengehalt, iso-Buten enthält.

4. Gemisch nach Anspruch 3, **dadurch gekennzeichnet, dass** das Kohlenwasserstoffgemisch 3 Gew.-% oder weniger, bezogen auf den Gesamtbutengehalt, iso-Buten enthält.

5. Gemisch nach einem der Ansprüche 2 bis 4, **dadurch gekennzeichnet, dass** der Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält.

6. Gemisch nach einem der Ansprüche 4 oder 5,**dadurch gekennzeichnet, dass** die Hydroformylierung in Gegenwart eines Cobalt-Katalysators erfolgt.

7. Verwendung eines Gemischs nach einem der vorhergehenden Ansprüche als weichmacher für Formmassen, insbesondere Formmas sen auf PVC-Basis.

8. Gemisch isomerer Nonanole, das
beim Verestern mit Adipinsäure zu einem Gemisch von Diestern der Adipinsäure führt, in dessen in CDCl₃ aufgenommenem ¹H-NMR-Spektrum das Verhältnis der Fläche unter den Resonanzsignalen bei chemischen Verschiebungen im Bereich von 1,0 bis 2,0 ppm gegen TMS zu der Fläche unter den Resonanzsignalen bei chemischen Verschiebungen im Bereich von 0,5 bis 1,0 ppm gegen TMS zwischen 1,20 und 5,00 liegt,
oder
beim Verestern mit Phthalsäure zu einem Gemisch von Diestern der Phthalsäure führt, in dessen in CDCl₃ aufgenommenem ¹H-NMR-Spektrum das Verhältnis der Fläche unter den Resonanzsignalen bei chemischen Verschiebungen im Bereich von 1,1 bis 3,0 ppm gegen TMS zu der Fläche unter den Resonanzsignalen bei chemischen Verschiebungen im Bereich von 0,5 bis 1,1 ppm gegen TMS zwischen 1,00 und 4,00 liegt.

9. Gemisch isomerer Nonoanole, erhältlich durch Hydroformylierung eines Gemisches isomerer Octene in Gegenwart eines Kobalt-Katalysators und Hydrierung des Hydroformylierungsproduktes, wobei das Gemisch isomerer Octene durch Inkontaktbringen eines Butene enthaltenden Kohlenwasserstoffgemisches, das 5 Gew.-% oder weniges, bezogen auf den gesanutbutengehalt, Isobuten enthält, mit einem Nickeloxid enthaltenden heterogenen Katalysator erhältlich ist.

10. Gemisch isomerer Nonanole nach Anspruch 9, **dadurch gekennzeichnet, dass** der heterogene Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminium-oxid und als Rest Siliciumdioxid enthält.

## Claims

1. A mixture of isomeric nonanol diesters of a dicarboxylic acid, where the material has been selected from the class consisting of
a mixture of diesters of adipic acid, in the ¹H NMR spectrum of which, observed in CDCl₃, the ratio of the area under the resonance signals at chemical shifts in the range from 1.0 to 2.0 ppm with respect to TMS to the area under the resonance signals at chemical shifts in the range from 0.5 to 1.0 ppm with respect to TMS is from 1.20 to 5.00
or
a mixture of diesters of phthalic acid, wherein, in the ¹H NMR spectrum of the same, observed in CDCl₃, the ratio of the area under the resonance signals at chemical shifts in the range from 1.1 to 3.0 ppm with respect to TMS to the area under the resonance signals at chemical shifts in the range from 0.5 to 1.1 ppm with respect to TMS is from 1.00 to 4.00.

2. A mixture as claimed in claim 1, obtainable by esterifying, with a dicarboxylic acid, selected from adipic acid and phthalic acid or a derivative thereof, an isomeric nonanols mixture obtainable by hydroformylating and hydrogenating an isomeric octenes mixture, where the isomeric octenes mixture is obtainable by bringing a butene-containing hydrocarbon mixture into contact with a heterogeneous catalyst comprising nickel oxide.

3. A mixture as claimed in claim 2, wherein the hydrocarbon mixture comprises 5% by weight or less of isobutene, based on the total butene content.

4. A mixture as claimed in claim 3, wherein the hydrocarbon mixture comprises 3% by weight or less of isobutene, based on the total butene content.

5. A mixture as claimed in any one of claims 2 to 4, wherein the catalyst comprises, as significant active constituents, from 10 to 70% by weight of nickel oxide, from 5 to 30% by weight of titanium dioxide and/or zirconium dioxide and from 0 to 20% by weight of aluminum oxide, and the remainder is silicon dioxide.

6. A mixture as claimed in either one of claims 4 and 5, wherein the hydroformylation takes place in the presence of a cobalt catalyst.

7. The use of a mixture as claimed in any one of the preceding claims as a plasticizer for molding compositions, in particular PVC-based molding compositions.

8. A mixture of isomeric nonanols which
upon esterification with adipic acid yields a mixture of diesters of adipic acid in the ¹H NMR spectrum of which, observed in CDCl₃, the ratio of the area under the resonance signals at chemical shifts in the range from 1.0 to 2.0 ppm with respect to TMS to the area under the resonance signals at chemical shifts in the range from 0.5 to 1.0 ppm with respect to TMS is from 1.20 to 5.00
or
upon esterification with phthalic acid yields a mixture of diesters of phthalic acid, in the ¹H NMR spectrum of which, observed in CDCl₃, the ratio of the area under the resonance signals at chemical shifts in the range from 1.1 to 3.0 ppm with respect to TMS to the area under the resonance signals at chemical shifts in the range from 0.5 to 1.1 ppm with respect to TMS is from 1.00 to 4.00.

9. A mixture of isomeric nonanols, obtainable by hydroformylating an isomeric octenes mixture in the presence of a cobalt catalyst and hydrogenating the hydroformylation product, where the isomeric octenes mixture is obtainable by bringing a butene-containing hydrocarbon mixture which comprises 5% by weight or less of isobutene, based on the total butene content, into contact with a heterogeneous catalyst comprising nickel oxide.

10. A mixture of isomeric nonanols as claimed in claim 9, wherein the heterogeneous catalyst comprises, as significant active constituents, from 10 to 70% by weight of nickel oxide, from 5 to 30% by weight of titanium dioxide and/or zirconium dioxide and from 0 to 20% by weight of aluminium oxide, and the remainder is silicon dioxide.

## Revendications

1. Mélange de diesters d'un acide dicarboxylique avec des isomères de nonanols, choisi parmi
un mélange de diesters de l'acide adipique dans le spectre RMN-¹H enregistré dans le CDCl₃ duquel le rapport de la surface située sous les signaux de résonance aux déplacements chimiques compris entre 1,0 et 2,0 ppm par rapport au TMS sur la surface située sous les signaux de résonance aux déplacements chimiques compris entre 0,5 et 1,0 ppm par rapport au TMS, est compris entre 1,20 et 5,00.
ou
un mélange de diesters de l'acide phtalique dans le spectre RMN-¹H enregistré dans le CDCl₃ duquel le rapport de la surface située sous les signaux de résonance aux déplacements chimiques compris entre 1,1 et 3,0 ppm par rapport au TMS sur la surface située sous les signaux de résonance aux déplacements chimiques compris entre 0,5 et 1,1 ppm par rapport au TMS, est compris entre 1,00 et 4,00.

2. Mélange selon la revendication 1, réalisable par estérification d'un mélange de nonanols isomères réalisable par hydroformylation et hydrogénation d'un mélange d'octènes isomères, avec un acide dicarboxylique choisi parmi l'acide adipique et l'acide phtalique ou un dérivé de ces derniers, le mélange d'octènes isomères étant réalisable par mise en contact d'un mélange d'hydrocarbures contenant des butènes avec un catalyseur hétérogène contenant de l'oxyde de nickel.

3. Mélange selon la revendication 2, **caractérisé en ce que** le mélange d'hydrocarbures contient 5% en poids ou moins d'isobutène par rapport à la teneur totale en butène.

4. Mélange selon la revendication 3, **caractérisé en ce que** le mélange d'hydrocarbures contient 3% en poids ou moins d'isobutène par rapport à la teneur totale en butène.

5. Mélange selon l'une des revendications 2 à 4, **caractérisé en ce que** le catalyseur contient comme composants actifs principaux 10 à 70% en poids d'oxyde de nickel, 5 à 30% en poids de dioxyde de titane et/ou de dioxyde de zirconium, 0 à 20% en poids d'oxyde d'aluminium et du dioxyde de silicium pour le reste.

6. Mélange selon l'une des revendications 4 ou 5, **caractérisé en ce que** l'hydroformylation s'effectue en présence d'un catalyseur au cobalt.

7. Utilisation d'un mélange selon l'une quelconque des revendications précédentes comme plastifiant pour les matières à mouler, en particulier pour les matières à mouler à base de PVC.

8. Mélange de nonanols isomères qui
au cours de l'estérification avec l'acide adipique donne un mélange de diesters de l'acide adipique dans le spectre RMN-¹H enregistré dans le CDCl₃ duquel le rapport entre la surface située sous les signaux de résonance aux déplacements chimiques compris entre 1,0 et 2,0 ppm par rapport au TMS et la surface située sous les signaux de résonance aux déplacements chimiques compris entre 0,5 et 1,0, ppm par rapport au TMS est compris entre 1,20 et 5,00,
ou
au cours de l'estérification avec l'acide phtalique donne un mélange de diesters de l'acide phtalique dans le spectre RMN-¹H enregistré dans le CDCl₃ duquel le rapport entre la surface située sous les signaux de résonance aux déplacements chimiques compris entre 1,1 et 3,0 ppm par rapport au TMS et la surface située sous les signaux de résonance aux déplacements chimiques compris entre 0,5 et 1,1 ppm par rapport au TMS est compris entre 1,00 et 4,00.

9. Mélange de nonanols isomères réalisable par hydroformylation d'un mélange d'octènes isomères en présence d'un catalyseur au cobalt et hydrogénation du produit d'hydroformylation, le mélange d'octènes isomères étant réalisable par mise en contact d'un mélange d'hydrocarbures contenant des butènes et 5% en poids ou moins d'isobutène par rapport à la teneur totale en butène, avec un catalyseur hétérogène contenant de l'oxyde de nickel.

10. Mélange de nonanols isomères selon la revendication 9, **caractérisé en ce que** le catalyseur hétérogène contient comme composants actifs principaux 10 à 70% en poids d'oxyde de nickel, 5 à 30% en poids de dioxyde de titane et/ou de dioxyde de zirconium, 0 à 20% en poids d'oxyde d'aluminium et du dioxyde de silicium pour le reste.
